# EUROPEAN PATENT APPLICATION

(11) **EP 4 790 149 A1**
(43) Date of publication of application: **12.08.2026**
(21) Application number: 24874815.4
(22) Date of filing: 17.05.2024
(51) Int. Cl.: C12Q 1/6886, C12Q 1/6869, G16B 30/00

(54) **APPARATUS AND METHOD FOR EARLY DISEASE DETECTION BASED ON HIGH-DEPTH CELL-FREE NUCLEIC ACID SEQUENCING**

(30) Priority: 06.10.2023 KR 20230133352; 25.01.2024 KR 20240011611
(71) Applicant: GENOME4ME, INC., Seoul 08826 (KR)
(72) Inventor: BAEK, Daehyun, Seoul 08826 (KR); AHN, Junhak, Seoul 08826 (KR); YUN, Jaeung, Seoul 08826 (KR); JEON, Hyeonseong, Seoul 08826 (KR)
(74) Representative: Laine IP Oy
(86) International application number: PCT/KR2024/006734
(87) International publication number: WO 2025/075256

(57) **Abstract**

The present invention relates to an apparatus for early disease detection based on high-depth cell-free nucleic acid sequencing, and may comprise: a library production part for producing, on the basis of cell-free nucleic acids extracted from blood, a library set including a whole genome sequencing library, a whole epigenome sequencing library, a transcriptome sequencing library, and an epi-transcriptome sequencing library; a sequencing part for generating high-depth cell-free nucleic acid sequencing data by sequencing the library set; a signal calculation part which maps the high-depth cell-free nucleic acid sequencing data to a reference genome so as to specify the position, on the genome, of the high-depth cell-free nucleic acid sequencing data, and which calculates at least one disease-specific signal value on the basis of the mapped high-depth cell-free nucleic acid sequencing data; a prediction signal value generation part for generating a disease prediction signal value by processing the at least one disease-specific signal value; and a prediction model generation part for generating a disease prediction model, which detects whether a disease occurs and the type of disease for input blood, by using labeling information related to blood and the disease prediction signal value.

## Description

### TECHNICAL FIELD

The present invention relates to a disease detection technology based on high-depth nucleic acid sequencing, and more particularly to a technology that is capable of the early detection of a disease based on high-depth sequencing of cell-free nucleic acids.

### BACKGROUND ART

Even in modern times with advanced medicine, most human deaths are caused by diseases. According to 2019 statistics, 88% of human deaths worldwide are due to diseases. Specifically, non-communicable diseases account for 74%, infectious diseases account for 14% of human deaths, and non-communicable diseases such as cancer and heart disease account for 51% of human deaths (https://ourworldindata.org).

One of the main reasons for death from diseases is due to the failure to recognize the disease early. For example, cancer, which ranks first and second among the causes of death worldwide, is discovered in the advanced stages of most cancer patients, making it very difficult to treat, and many patients die within two years. This high cancer mortality rate is not only a problem with treatment methods, but is also closely related to the lack of methods for detecting cancer early.

The early detection of disease has been consistently reported to significantly increase the patient's survival rate. According to a 2022 report by Cancer Research UK, there is a very large difference in the survival rates when cancer is detected early and when it is detected at a late stage. The survival rates of lung cancer, colon cancer and breast cancer are high at about 60%, 90% and 100%, respectively, when cancer is detected early, but very low at about 10%, 10% and 30%, respectively, when cancer is detected at a late stage. Particularly, in cases such as pancreatic cancer where there are few symptoms in the early stage or where there is no effective method for early cancer detection, cancer is often detected at a late stage, and most cases lead to death.

Although it may vary depending on the type of disease, early detection is quite difficult. Currently, in order to successfully detect diseases early, patients must voluntarily perform detailed health checkups for various diseases (even if they are painless or in a mild state), and even if they perform health checkups, the diseases may not be successfully detected. For example, in the current clinical setting, cancer is diagnosed primarily through questioning and physical examination, and if there is any suspicion, X-ray examinations and endoscopic examinations are performed, and finally, cancer is confirmed through tissue examination. However, in order to detect cancer using clinical examination methods, a significant number of cancer cells must be present, and the diameter of the cancer must be larger than a certain size. More than half of cancers of this size are generally found in a metastatic state. In addition, cancer detection technology in the current clinical stage is limited to detecting specific types of cancer, and since individual cancer-specific detection tests must be performed independently multiple times, cancer detection becomes difficult and cumbersome. Only a small number of patients with early cancer who have no or very few symptoms voluntarily and preemptively undergo these multiple independent cancer detection tests.

The development of accessible and easy-to-use disease detection tests, such as blood tests, may increase the frequency of voluntary disease screening and thus increase the sensitivity of early disease detection. A number of previous research results have suggested that analyzing cell-free nucleic acid sequencing data from blood can detect specific diseases. These attempts have been performed for a variety of diseases, including cancers such as colon, lung and breast cancers (Klein et al., 2022, Ann. Oncol.; Fairchild et al., 2023, Sci. Transl. Med.), rare diseases including Erdheim-Chester disease and Klippel-Trenaunay syndrome (Janku et al., 2014, Oncotarget; Palmieri et al., 2020, Vascular*.*), and immune diseases including systemic lupus erythematosus and rheumatoid arthritis (Truszewska et al., 2020, Lupus; Ding et al., 2022, Clin. Chem.; Zhou et al., 2023, PNAS; Plant et al., 2016, Arthritis Rheumatol.), chronic diseases including hypertension (Brusca et al., 2022, Circulation), cardiac diseases including chronic heart disease (Zemmour et al., 2018, Nat. Commun.), metabolic diseases including fatty liver disease and diabetes (Karlas et al., 2017, J. Transl. Med.; Lehmann-Werman et al., 2016, PNAS), and infectious diseases including infectious pneumonia and invasive fungal infections (Langelier et al., 2020, Am. J. Respir. Crit. Car. Med.; Hong et al., 2018, Diagn. Microbiol. Infect. Dis.).

Recently, attempts have been reported to extract disease detection markers from cell-free nucleic acid sequencing data of patient blood to detect diseases early. A recent cancer study (Klein et al., 2022, Ann. Oncol.) reported that panel sequencing of the epigenome library of cell-free nucleic acids could detect stage 1 cancer with a sensitivity of about 17% at a specificity of 99.5%. However, this is a considerably low numerical value compared to the detection sensitivity of stage 3 and 4 cancer in the same study (about 70% at a specificity of 99.5%). Another study (Nicholson et al., 2023, Lancet) also reported a low detection rate of about 24% at a specificity of 98% in stage 1 cancer. These previous research results show the possibility of early disease detection, but at the same time, they strongly suggest the need for the development of highly accurate early disease detection test methods.

Meanwhile, research results for the early detection of diseases using whole genome sequencing rather than panel sequencing have been recently reported, which sequence cell-free nucleic acids in blood at a low depth of 1 to 5 (Cristiano et al., 2019, Nature; Mouliere et al., 2019, Sci. Transl. Med.; Wan et al., 2022, Nat. Commun.; An et al., 2023, Nat. Commun.; Bruhm et al., 2023, Nat. Genet.; Budhraja et al., 2023, Sci. Transl. Med.). Depth refers to the average number of sequencing reads that have information on a specific location on the genome. The higher the depth, the more sequencing reads have information on a specific location on the genome, which can increase the amount and accuracy of genome information that can be obtained. This suggests that utilizing high-depth cell-free nucleic acid sequencing to detect diseases could lead to more accurate early disease detection than existing cell-free nucleic acid-based cancer detection methods. Therefore, a method for early disease detection based on such high-depth cell-free nucleic acid sequencing is needed.

### DISCLOSURE

### TECHNICAL PROBLEM

One embodiment of the present invention relates to an apparatus and method for the early detection of a disease based on high-depth cell-free nucleic acid sequencing, which can obtain high-depth cell-free nucleic acid sequencing data for cell-free nucleic acid extracted from blood.

One embodiment of the present invention relates to an apparatus and method for the early detection of a disease based on high-depth cell-free nucleic acid sequencing, which can detect a disease by calculating a disease-specific signal value based on high-depth cell-free nucleic acid sequencing data mapped to a reference genome.

One embodiment of the present invention relates to an apparatus and method for the early detection of a disease based on high-depth cell-free nucleic acid sequencing, which can detect whether a subject has a disease and the type of a disease using only free nucleic acids extracted from the blood of the subject, by using disease prediction signal values obtained by high-depth sequencing of free nucleic acids extracted from the blood.

One embodiment of the present invention relates to a method for the early detection of a disease using only free nucleic acids extracted from the blood of a subject by using disease prediction signal values obtained by highly deep sequencing of free nucleic acids extracted from the blood.

### TECHNICAL SOLUTION

The apparatus for early disease detection based on high-depth cell-free nucleic acid sequencing according to an embodiment of the present invention may include a library production part configured to produce a library set including a whole genome sequencing library, a whole epigenome sequencing library and a transcriptome sequencing library based on cell-free nucleic acids extracted from blood; a sequencing part configured to generate high-depth cell-free nucleic acid sequencing data by sequencing the library set at a high depth of 10 or more; a signal calculation part configured to map the high-depth cell-free nucleic acid sequencing data to a reference genome so as to specify a location of the high-depth cell-free nucleic acid sequencing data on a genome, and calculate at least one disease-specific signal value based on the mapped high-depth cell-free nucleic acid sequencing data; a prediction signal value generation part configured to generate a disease prediction signal value by processing the at least one disease-specific signal value; and a prediction model generation part configured to generate a disease prediction model that detects whether a disease occurs and the type of a disease for input blood by using labeling information related to blood and the disease prediction signal value.

The blood may be extracted from individuals without disease, individuals with a history of a specific disease but currently without disease, patients in the early, middle and late stages of a specific disease, and patients with a relapse of a specific disease.

The signal calculation part may calculate the disease-specific signal value based on one or more of a DNA signal value, a DNA modification signal value, an RNA signal value and an RNA modification signal value.

The prediction signal value generation part may process a plurality of disease-specific signal values into vector data to generate the disease prediction signal value.

The prediction signal value generation part may generate the disease prediction signal value according to the type of a target disease.

The apparatus may further include a disease prediction part configured to receive blood data extracted from a subject and detects whether the subject has a disease and the type of a disease through the disease prediction model.

The sequencing part may utilize two or more sequencers to sequence a library set.

The method for detecting a disease based on high-depth cell-free nucleic acid sequencing according to an embodiment of the present invention may include producing a library set including a whole genome sequencing library, a whole epigenome sequencing library and a transcriptome sequencing library based on cell-free nucleic acids extracted from blood; generating high-depth cell-free nucleic acid sequencing data by sequencing the library set; mapping the high-depth cell-free nucleic acid sequencing data to a reference genome so as to specify a location of the high-depth cell-free nucleic acid sequencing data on a genome, and calculating at least one disease-specific signal value based on the mapped high-depth cell-free nucleic acid sequencing data; generating a disease prediction signal value by processing the at least one disease-specific signal value; generating a disease prediction model by using labeling information related to blood and the disease prediction signal value and detecting whether a subject has developed a disease and the type of disease by using the disease prediction model and blood data extracted from the subject.

### ADVANTAGEOUS EFFECT

The disclosed technology may have the following effects. However, this does not mean that a specific embodiment must include all or only the following effects, and thus, the scope of the disclosed technology should not be construed as being limited thereby.

A disease detection apparatus and method based on high-depth cell-free nucleic acid sequencing according to one embodiment of the present invention can obtain high-depth cell-free nucleic acid sequencing data for cell-free nucleic acid extracted from blood.

A disease detection apparatus and method based on high-depth cell-free nucleic acid sequencing according to one embodiment of the present invention can detect general diseases at an early stage by calculating disease-specific signal values based on high-depth cell-free nucleic acid sequencing data mapped to a reference genome.

A disease detection apparatus and method based on high-depth cell-free nucleic acid sequencing according to one embodiment of the present invention can detect the early onset of a disease in a subject using only cell-free nucleic acids included in the blood of the subject by using a disease prediction signal value obtained by high-depth sequencing of cell-free nucleic acids extracted from blood.

A disease detection apparatus and method based on high-depth cell-free nucleic acid sequencing according to one embodiment of the present invention can distinguish the type of disease at an early stage using only cell-free nucleic acids included in the blood of a subject by using a disease prediction signal value obtained by high-depth sequencing of cell-free nucleic acids extracted from blood.

### DESCRIPTION OF THE DRAWINGS

FIG. 1 is a diagram for describing a disease early detection system based on high-depth cell-free nucleic acid sequencing according to one embodiment of the present invention.
FIG. 2 is a diagram for describing the physical configuration of a disease early detection apparatus based on high-depth cell-free nucleic acid sequencing according to one embodiment of the present invention.
FIG. 3 is a diagram for describing the functional configuration of a disease early detection apparatus based on high-depth cell-free nucleic acid sequencing according to one embodiment of the present invention.
FIG. 4 is a diagram comparing high-depth whole genome sequencing with conventional whole genome sequencing.
FIG. 5 shows an example of cancer detection based on high-depth cell-free nucleic acid sequencing according to one embodiment of the present invention.
FIG. 6 is a diagram for describing a method for classifying cancer patients based on cancer-specific signal values according to one embodiment of the present invention.
FIG. 7 shows specific examples of disease-specific signal values for generating disease prediction signal values according to one embodiment of the present invention.
FIG. 8 is a diagram for describing a disease prediction signal value according to one embodiment of the present invention.
FIG. 9 is a diagram for describing the sequence of a disease early detection method based on high-depth cell-free nucleic acid sequencing according to one embodiment of the present invention.

### MODES FOR INVENTION

The description of the present invention is only an embodiment for structural and functional explanation, and thus, the scope of the rights of the present invention should not be construed as being limited by the embodiments described in the text. That is, since the embodiments can be variously modified and can have various forms, the scope of the rights of the present invention should be understood to include equivalents that can realize the technical idea. In addition, the objects or effects presented in the present invention do not mean that a specific embodiment must include all of the same or only such effects, and thus, the scope of the rights of the present invention should not be understood as being limited thereby.

Meanwhile, the meanings of the terms described in the present application should be understood as follows.

Terms such as "first", "second" and the like are intended to distinguish one component from another, and the scope of the rights should not be limited by these terms. For example, the first component may be referred to as the second component, and similarly, the second component may also be referred to as the first component.

When it is mentioned that a component is "connected" to another component, it should be understood that it may be directly connected to that other component, but there may also be other components in between. On the other hand, when it is mentioned that a component is "directly connected" to another component, it should be understood that there are no other components in between. Meanwhile, other expressions that describe the relationship between components, such as "between" and "directly between" or "adjacent to" and "directly adjacent to", should be interpreted similarly.

A singular expression should be understood to include the plural expression unless the context clearly indicates otherwise, and terms such as "include" or "have" should be understood to specify the presence of a feature, number, step, operation, component, part or combination thereof, but not to exclude the possibility of the presence or addition of one or more other features, numbers, steps, operations, components, parts or combinations thereof.

In each step, the identifiers (e.g., a, b, c, etc.) are used for the convenience of explanation and do not describe the order of each step, and each step may occur in a different order than stated unless the context clearly indicates a specific order. That is, each step may occur in the same order as stated, may be performed substantially simultaneously, or may be performed in the opposite order.

The present invention may be implemented as a computer-readable code on a computer-readable recording medium, and the computer-readable recording medium includes all types of recording devices that store data that can be read by a computer system. Examples of the computer-readable recording medium include a ROM (Read Only Memory), a RAM (Random Access Memory), a CD-ROM, a magnetic tape, a floppy disk, an optical data storage device and the like. In addition, the computer-readable recording medium may be distributed over computer systems connected to a network such that the computer-readable code can be stored and executed in a distributed manner.

All terms used herein, unless otherwise defined, have the same meanings as commonly understood by a person of ordinary skill in the art to which the present invention pertains. Terms defined in commonly used dictionaries should be interpreted as having meanings consistent with the contextual meanings of the relevant art, and shall not be interpreted as having ideal or overly formal meanings unless explicitly defined in the present application.

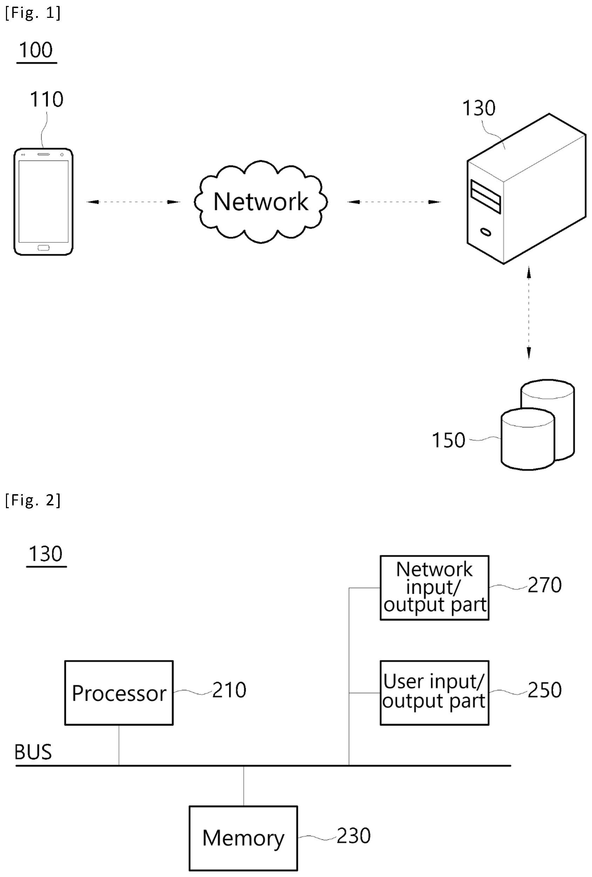

FIG. 1 is a diagram for describing a disease detection system 100 based on high-depth cell-free nucleic acid sequencing according to one embodiment of the present invention.

Referring to FIG. 1, the disease detection system 100 based on high-depth cell-free nucleic acid sequencing may include a user terminal 110, a disease detection apparatus based on high-depth cell-free nucleic acid sequencing 130 and a database 150.

The user terminal 110 may be implemented as a smart phone or a wearable device capable of checking the data generated based on the operations of generating a disease early detection apparatus configured to produce a library set based on cell-free nucleic acids through a disease detection apparatus 130 based on high-depth cell-free nucleic acid sequencing, generate high-depth cell-free nucleic acid sequencing data for the library set, generate a disease-specific signal value and a disease prediction signal value based on the mapped high-depth cell-free nucleic acid sequencing data, and generate a disease prediction model that predicts whether a disease occurs and the type of a disease in the blood of a subject, and analyze the data and metadata analyzed therefrom, but is not necessarily limited thereto and may also be implemented as various devices such as a tablet PC. The user terminal 110 may be connected to the disease detection apparatus 130 based on high-depth cell-free nucleic acid sequencing through a network, and a plurality of user terminals 110 may be simultaneously connected to the disease detection apparatus 130 based on high-depth cell-free nucleic acid sequencing.

The disease detection apparatus 130 based on high-depthcell-free nucleic acid sequencing may be implemented as a server corresponding to a computer or program that sequentially performs the operations of generating a disease early detection apparatus. The disease detection apparatus 130 based on high-depth cell-free nucleic acid sequencing may be wirelessly connected to a user terminal 110 through Bluetooth, WiFi, a communication network and the like, and may exchange data with the user terminal 110 through the network.

The disease detection apparatus 130 based on high-depth cell-free nucleic acid sequencing may be provided by being included in a computer-readable recording medium by tangibly implementing a program of commands for implementing the same. In other words, the disease detection apparatus 130 based on high-depth cell-free nucleic acid sequencing may be implemented in the form of program commands that can be executed through various computer means, and may be recorded in a computer-readable recording medium. In addition, the disease detection apparatus 130 based on high-depth cell-free nucleic acid sequencing may consist of a computer program that sequentially performs the generating operations of a disease early detection apparatus, and the computer program may be stored in a computer-readable recording medium.

The database 150 may correspond to a storage device that stores various pieces of information generated through the generating operations of a disease early detection apparatus.

FIG. 2 is a diagram showing the physical configuration of a disease detection apparatus 130 based on high-depth cell-free nucleic acid sequencing according to one embodiment.

Referring to FIG. 2, the disease detection apparatus 130 based on high-depth cell-free nucleic acid sequencing may be implemented by including a processor 210, a memory 230, a user input/output part 250 and a network input/output part 270.

The processor 210 may execute a procedure for performing the generating operations of a disease early detection apparatus, manage a memory 230 that is read or written throughout the process, and schedule a synchronization time between a volatile memory and a non-volatile memory in the memory 230. The processor 210 may control the overall operation of the disease detection apparatus 130 based on high-depth cell-free nucleic acid sequencing, and is electrically connected to the memory 230, the user input/output part 250 and the network input/output part 270 to control data flow therebetween. The processor 210 may be implemented as a CPU (Central Processing Unit) or a GPU (Graphics Processing Unit) of the disease detection apparatus 130 based on high-depth cell-free nucleic acid sequencing.

The memory 230 may include an auxiliary memory device implemented as a non-volatile memory such as an SSD (Solid State Drive) or an HDD (Hard Disk Drive) and used to store all data required for a disease detection apparatus 130 based on high-depth cell-free nucleic acid sequencing, and may include a main memory device implemented as a volatile memory such as RAM.

The user input/output part 250 may include an environment for receiving user input and an environment for outputting specific information to the user. For example, the user input/output part 250 may include an input device including an adapter such as a touch pad, a touch screen, a visual keyboard or a pointing device, and an output device including an adapter such as a monitor or a touch screen. In one embodiment, the user input/output part 250 may correspond to a computing device that is connected via a remote connection, and in such a case, the disease detection apparatus 130 based on high-depth cell-free nucleic acid sequencing may be performed as a server.

The network input/output part 270 includes an environment for connecting to an external device or system via a network, and may include an adapter for communication such as, for example, a Local Area Network (LAN), a Metropolitan Area Network (MAN), a Wide Area Network (WAN) and a Value Added Network (VAN).

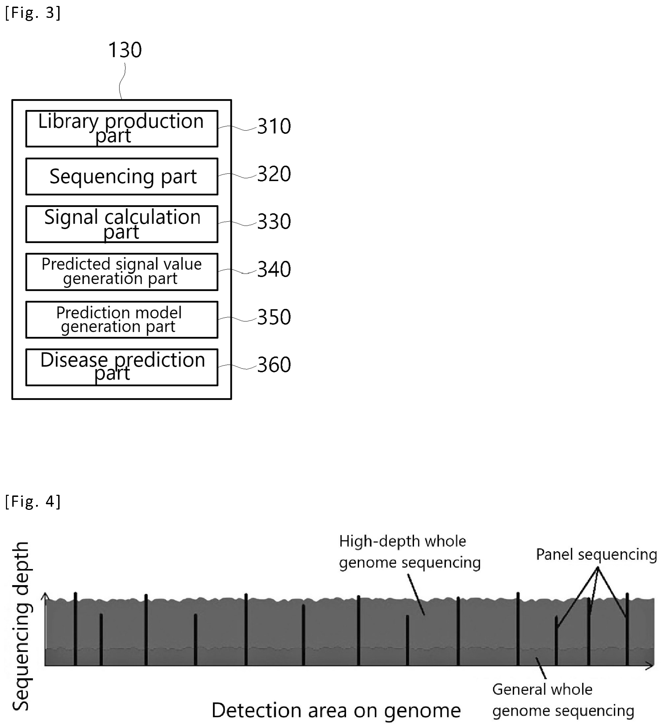

FIG. 3 is a diagram for explaining the functional configuration of a disease detection apparatus 130 based on high-depth cell-free nucleic acid sequencing according to one embodiment of the present invention. Referring to FIG. 3, the disease detection apparatus 130 based on high-depth cell-free nucleic acid sequencing may include a library production part 310, a sequencing part 320, a signal calculation part 330, a prediction signal value generation part 340, a prediction model generation part 350 and a disease prediction part 360.

The library production part 310 may produce a library set including a whole genome sequencing library, a whole epigenome sequencing library, a transcriptome sequencing library and an epitranscriptome sequencing library based on cell-free nucleic acids extracted from blood.

Here, cell-free nucleic acids may be extracted from plasma separated from blood. Cell-free nucleic acids may include cell-free DNA and cell-free RNA, and cell-free DNA and cell-free RNA may include exosome DNA and exosome RNA, respectively. For example, cell-free nucleic acids may be extracted via the Circulating Nucleic Acid Kit.

Blood may be extracted from individuals without a disease, individuals who have a history of a specific disease but are currently without a disease, patients in the early, middle and late stages of a specific disease, and patients with a relapsed disease. In addition to distinguishing between individuals with and without disease, blood characteristics may differ depending on the degree of disease progression or whether treatment has been administered.

The library production part 310 may produce a library set for the corresponding cell-free nucleic acids extracted from blood through an adapter ligation step and a library quantification and size confirmation step by using a library production kit. In addition, the library production part 310 may produce a whole genome bisulfite sequencing library as one method of producing a whole epigenome sequencing library based on the cell-free nucleic acid. For example, the library production part 310 may produce a library set by using the EpiNext High-Sensitivity DNA Library Preparation Kit, the EZ DNA Methylation-Lightning MagPrep Kit or the EpiNext Post-Bisulfite DNA Library Preparation Kit. In addition, the library production part 310 may produce a transcriptome bisulfite sequencing library as one method of producing an epitranscriptome sequencing library based on the cell-free nucleic acid. For example, the library production part 310 may produce a library set by using the AMPINEXT 5-mC RNA Bisulfite-Seq Easy Kit or the EpiNext RNA Bisulfite-Seq Kit.

FIG. 4 is a diagram comparing high-depth whole genome sequencing with conventional whole genome sequencing.

Referring to FIG. 4, the sequencing part 320 may generate high-depth genome sequencing data by sequencing a library set. The sequencing part 320 may generate high-depth genome sequencing data with a higher depth than general genome sequencing for the entire genome region. Compared to panel sequencing that sequences only a specific genome region, the sequencing part 320 may sequence the entire genome region at a high depth to generate sequencing data. Through this, the disease detection apparatus 130 based on high-depth cell-free nucleic acid sequencing according to the present invention may detect a disease with high accuracy based on high-depth sequencing data for the entire genome region.

In one embodiment, the sequencing part 320 may sequence the library set at a high depth of 10 or more. Here, the depth means the average number of sequencing reads having specific location information on the genome. As the depth increases, the number of sequencing reads having specific location information on the genome increases, and thus the amount and accuracy of genome information that can be obtained may increase.

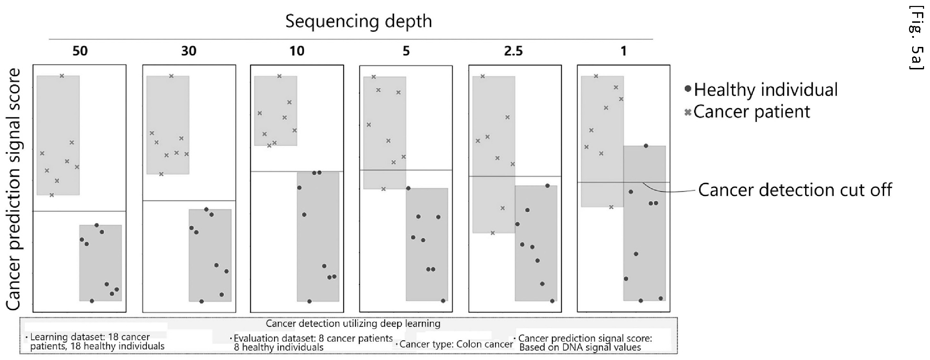

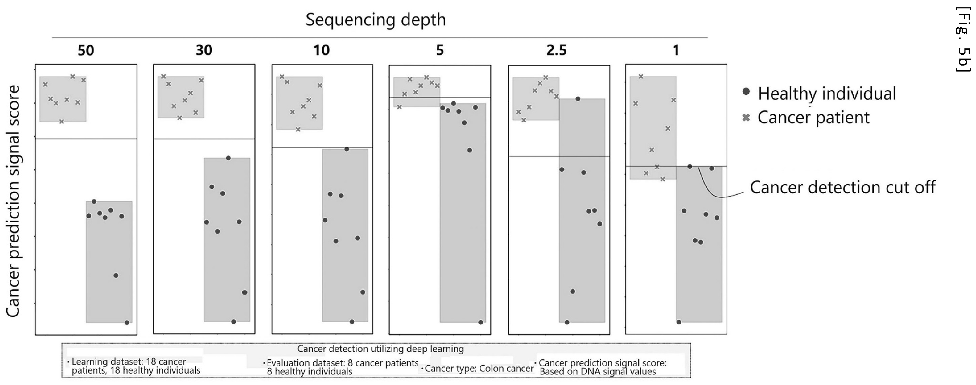

FIG. 5 shows the results of predicting cancer by extracting cancer-specific signals by whole genome sequencing of cell-free nucleic acids in blood at each sequencing depth. The present invention may be applied not only to cancer as in the example of FIG. 5, but also to other diseases.

FIG. 5A shows the prediction results when a cancer prediction signal value is generated by using a DNA signal value.

The dataset used for learning is data from 18 cancer patients and 18 healthy individuals, and the dataset used to evaluate the learning results is data from 8 cancer patients and 8 healthy individuals. The predicted cancer is colon cancer.

As a result of the experiment, it was confirmed that when the whole genome sequencing data with a high depth of 10 or more of cell-free nucleic acid in blood was utilized according to the present invention, that is, when the sequencing data with a depth of 10, 30 and 50 was utilized, the cancer prediction signal scores of cancer patients and healthy individuals were more clearly distinguished, and when the low-depth whole genome sequencing data with a depth of less than 10 was used, the boundary between the cancer prediction signal scores of cancer patients and healthy individuals was unclear.

FIG. 5B shows the prediction results when a cancer prediction signal value is generated by additionally using a DNA modification signal value in addition to a DNA signal value.

The inventors of the present invention tested colon cancer under the same conditions as in FIG. 5A, and it can be confirmed that when the whole genome sequencing of cell-free nucleic acids in the blood was performed at a high depth of 10 or more, the cancer prediction signal scores of cancer patients and healthy individuals were more clearly distinguished, as when only the DNA signal value was used.

In one embodiment, the sequencing part 320 may utilize one or more types of sequencers to sequence the library set. Examples of sequencers that can be utilized include sequencers from Illumina, MGI, Element Biosciences, Ultima Genomics, PacBio, Oxford Nanopore and the like. Additionally, various sequencers may be utilized without being limited by the length of the sequencing read or the sequencing technology (e.g., chain termination, ligation, fluorescence, single/paired-end, etc.).

The signal calculation part 330 may map the high-depth cell-free nucleic acid sequencing data to a reference genome to specify the location of the sequencing read on a genome. More specifically, the signal calculation part 330 may map the corresponding high-depth cell-free nucleic acid sequencing data to a suitable reference genome such that the generated high-depth cell-free nucleic acid sequencing data can be analyzed by specifying the location on the genome. That is, the signal calculation part 330 may specify the location such that the pre-generated high-depth cell-free nucleic acid sequencing data can be analyzed.

In one embodiment, the signal calculation part 330 may map high-depth cell-free nucleic acid sequencing data to a reference genome. High-depth cell-free nucleic acid sequencing data mapped to a reference genome may be stored as a file such as a Sequence Alignment Map (SAM), a Binary Alignment Map (BAM) or a Compressed Reference-oriented Alignment Map (CRAM). The reference genome to which the high-depth cell-free nucleic acid sequencing data is mapped is not limited to a human reference genome. In order to detect an infectious disease, the extent of the presence of a genome sequence of an infectious agent may be confirmed by mapping to a reference genome other than human (virus, bacteria, fungi, etc.). For example, in order to detect bacterial infectious pneumonia, high-depth cell-free nucleic acid sequencing data may be mapped to pneumococcus reference genome data.

The signal calculation part 330 may calculate at least one disease-specific signal value based on the mapped high-depth cell-free nucleic acid sequencing data. The signal calculation part 330 may analyze the high-depth cell-free nucleic acid sequencing data to generate a disease-specific signal value that is related to the occurrence of a disease. More specifically, the disease-specific signal value may show similar or different aspects depending on the type of a disease.

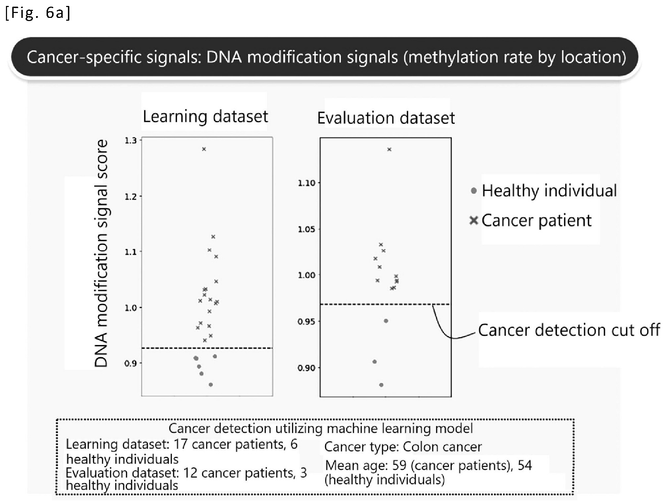

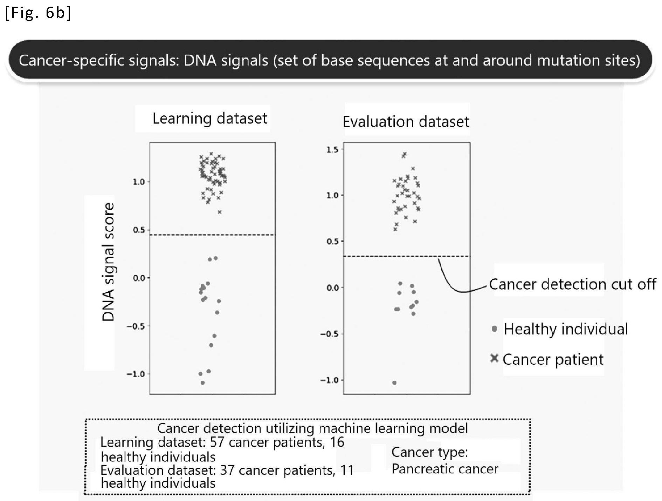

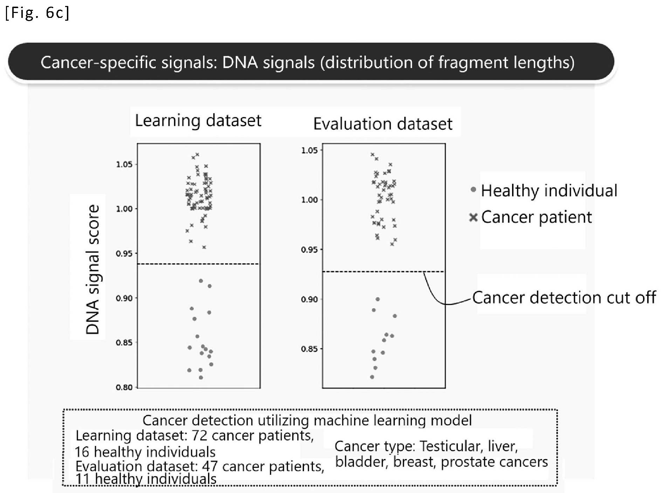

FIG. 6 is a diagram for explaining a method for classifying a subject based on a disease-specific signal value according to one embodiment of the present invention.

For example, in FIG. 6A, the DNA modification signal value (utilizing the degree of methylation modification by location), which is one of the disease-specific signal values, may be associated with colon cancer, and healthy individuals and cancer patients may be distinguished based on a specific cancer detection cut-off value.

For another example, in FIG. 6B, the DNA signal value (utilizing a set of base sequences around the mutation site), which is one of the cancer-specific signal values, may be associated with pancreatic cancer, and healthy individuals and cancer patients may be distinguished based on a specific cancer detection cut-off value.

As another example, in FIG. 6C, the DNA signal value (distribution of fragment length), which is one of the cancer-specific signal values, may be associated with testicular cancer, liver cancer, bladder cancer, breast cancer and prostate cancer, and healthy individuals and cancer patients may be distinguished based on a specific cancer detection cut off.

The signal calculation part 330 may generate a disease-specific signal value that can distinguish between a healthy individual and a diseased patient for a specific disease, and may generate a disease prediction signal value by combining one or more disease-specific signal values as described below, and may detect various types of diseases through the disease prediction signal value. This will be described in detail below.

In one embodiment, the signal calculation part 330 may calculate a disease-specific signal value based on a DNA signal value, a DNA modification signal value, an RNA signal value and an RNA modification signal value.

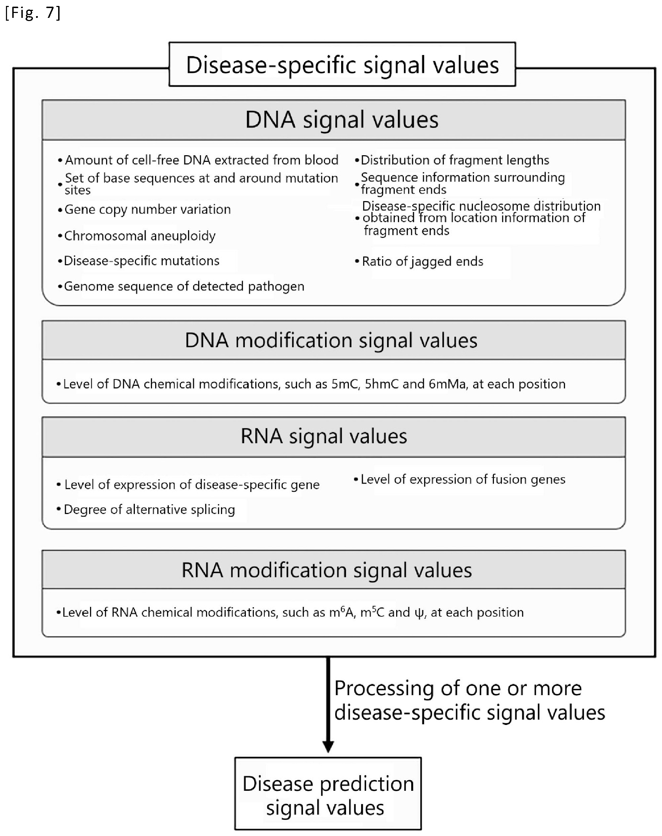

FIG. 7 shows examples of data for calculating the disease-specific signal values.

The signal calculation part 330 may calculate a DNA signal value by using high-depth whole-genome cell-free nucleic acid sequencing data sequenced from a whole genome sequencing library.

The DNA signal value may include one or more of an amount of cell-free DNA extracted from blood, a set of base sequences at and around a mutation site, a gene copy number variation, a disease-specific mutation, a genome sequence of a detected pathogen, a distribution of fragment lengths, sequence information around fragment ends, individual disease-specific nucleosome distributions obtained from location information at fragment ends and a ratio of jagged ends.

In more detail, the signal calculation part 330 filters previously known single nucleotide polymorphism (SNP) sites or filters low-quality mutation sites by considering base quality, mapping quality and depth on the sequencing read. A set of base sequences at the mutation site after filtering, a set of base sequences around the mutation site (e.g., 2X+1 consecutive bases from -X nt to +X nt of the mutation site), a set of sequencing read information at and around the mutation site, a correlation coefficient level comparing the sets of base sequences at the mutation site with a well-known disease-specific mutation reference (e.g., the mutational signature of COSMIC for cancer), and the presence or absence of a disease-specific associated mutation may be utilized to generate a DNA signal value. Previous studies have observed significant differences in mutation sites and surrounding base sequence sets between patients and non-patients in diseases such as cancer and clonal hematopoiesis (Bruhm et al., 2023, Nat. Genet.; Fairchild et al., 2023, Sci. Transl. Med.). The mutations include structural variations such as single base substitutions, doublet base substitutions, small insertions and deletions, large insertions and deletions, tandem duplications, inversions, fusions, translocations, chromoplexies and chromothripsis. In the case of structural variations, the breakpoint positions, which are discontinuous points on the genome of sequencing reads, and the base sequence sets around those positions may be used as DNA signal values.

The signal calculation part 330 may utilize a pathogen genome sequence detected from the high-depth whole-genome cell-free nucleic acid sequencing data to generate a DNA signal value. The pathogen may include pathogenic DNA viruses, bacteria, fungi and the like that can cause infectious diseases.

The signal calculation part 330 may utilize the level of gene copy number alteration and the level of chromosomal aneuploidy detected from high-depth whole-genome cell-free nucleic acid sequencing data to generate a DNA signal value.

The signal calculation part 330 may utilize fragment information obtained from high-depth cell-free nucleic acid sequencing data to generate a DNA signal value.

More specifically, the distribution of fragment lengths, the set of sequence information of Y consecutive bases around the fragment ends, and the individual disease-specific nucleosome distribution obtained from the location information of the fragment ends on the genome may be utilized to generate DNA signal values. Previous studies have observed significant differences in fragment signals between patients and non-patients in diseases such as cancer, systemic lupus erythematosus and fatty liver (Cristiano et al., 2019, Nature; Ding et al., 2022, Clin. Chem.; Zhou et al., 2023, PNAS; Karlas et al., 2017, J. Transl. Med.).

The signal calculation part 330 may utilize the amount of cell-free DNA extracted from blood in the library production part 310 to generate a DNA signal value.

More specifically, the amount of cell-free DNA may be determined by using electrophoresis or absorbance measurement of cell-free nucleic acids labeled with fluorescent markers. For example, the amount of cell-free DNA may be determined by utilizing the Agilent 2100 Bioanalyzer or the Invitrogen Qubit 4.0 fluorometer. Previous studies have observed significant differences in the amount of cell-free DNA between patients and non-patients in diseases such as cancer, systemic lupus erythematosus, rheumatoid arthritis, hypertension and heart disease (Chen et al., 2021, Sci. Rep.; Truszewska et al., 2020, Lupus; Hashimoto et al., 2016, Int. J. Rheum.; Jeong et al., 2015, Kidney. Res. Clin. Pract.; Antonatos et al., 2006, Ann. N. Y. Acad. Sci.).

The DNA modification signal value may include one or more position-specific modification levels of DNA chemical modification including 1mA, 1mHyp, 3mA, 3mHyp, 4mA, 6mA, 7mA, 7mHyp, 9mA, 9mHyp, 6hmA, m2A, ncm6A, xI, m2C, e3C, 3mC, εC, 4mC, 5caC, 5fC, 5hmC, 5mC, 1mG, 2mG, 3mG, m6G, 7mG, CEG, M22G, N(2),3-εG, 1,N(2)-εG, ADG, 8oxoG, dU, dhpdU, 5-NedU, DHdU, 5-NeOmdU, 5caU, dhpUra, 5hmU, 5fU, base J, diHT, 1mT, 3mT, 4-meT and putThy.

The signal calculation part 330 may calculate a DNA modification signal value by using high-depth whole genome sequencing data sequenced from a sequencing library specifically produced to detect specific chemical modifications of DNA (e.g., including 5mC, 5hmC, 6mA, etc.), or high-depth whole epigenome sequencing data based on a specific sequencer (e.g., sequencer from Oxford Nanopore) that can directly read DNA modification information from the whole genome sequencing library.

For example, in order to detect 5mC, which is a type of DNA methylation, a whole genome bisulfite sequencing library may be produced, and high-depth cell-free nucleic acid sequencing data may be utilized by sequencing the same with an Illumina sequencer, or a whole genome sequencing library may be utilized for high-depth cell-free nucleic acid sequencing data which is sequenced with an Oxford Nanopore sequencer.

In more detail, the signal calculation part 330 obtains DNA chemical modification sites and sites where DNA chemical modification does not occur from high-depth whole epigenome sequencing data or a whole genome sequencing library based on a specific sequencer, and filters out previously known single nucleotide polymorphism sites or removes inappropriate DNA modification sites by considering base quality, mapping quality, depth and the like on the sequencing read. Thereafter, the DNA chemical modification signals of a disease patient and a normal person are compared to select a DNA chemical modification region (differentially modified region, DMR) whose pattern is significantly different in the disease patient, and the level of DNA chemical modification at the corresponding site may be calculated as a DNA modification signal value. In addition, the signal calculation part 330 may reflect the level of DNA chemical modification at the corresponding site, sequencing read information and the like, in the DNA modification signal value. Previous studies have observed significant differences in methylation signals between patients and non-patients in diseases such as cancer, systemic lupus erythematosus, rheumatoid arthritis, hypertension, heart disease and fatty liver (Shen et al., 2018, Nature; Wang et al., 2022, Front. Immunol.; Plant et al., 2016, Arthritis Rheumatol.; Brusca et al., 2022, Circulation; Zemmour et al., 2018, Nat. Commun.; Yigit et al., 2018, Gut).

The signal calculation part 330 may calculate an RNA signal value by using high-depth transcriptome sequencing data sequenced from a transcriptome sequencing library.

The RNA signal value may include one or more of the expression level of an individual disease-specific gene, the expression level of a fusion gene and the degree of alternative splicing.

More specifically, the signal calculation part 330 may calculate an RNA signal value by removing low-quality sequencing reads by considering base quality, mapping quality, depth and the like on the sequencing reads and confirming whether there are sufficient sequencing reads that can support the expression of genes known to cause diseases. In addition, the signal calculation part 330 may utilize the gene expression level of a disease-specific gene, the expression level of a fusion gene, the type of expressed fusion gene and the degree of alternative splicing to calculate an RNA signal value. Alternative splicing may include one or more of intron retention, exon skipping, alternative 5' splicing, alternative 3' splicing, mutually exclusive splicing, alternative transcription initiation, and alternative polyadenylation. Previous studies have observed significant differences in the expression levels of disease-specific genes and fusion genes and the degree of alternative splicing between patients and non-patients in diseases such as cancer (Larson et al., 2021, Nat. Commun.; Hasegawa et al., 2021, Cancer Sci.; Zhu et al., 2021, Theranostics*.*).

The RNA modification signal value may include one or more of position-specific modification levels of RNA chemical modification including ms2m6A, io6A, m6t6A, hn6A, Ar(p), m1I, m1Am, m1Im, m2A, m62A, ms2i6A, ms2t6A, i6A, ms2hn6A, m6Am, m1A, m6A, I, g6A, m62Am, ac6A, ms2io6A, Am, im, t6A, m8A, m2,8A, ct6A, hm6A, f6A, ms2ct6A, ht6A, msms2i6A, m5Cm, m4C, m42C, m5C, f5C, m4Cm, m3C, m42Cm, ac4C, Cm, s2C, k2C, f5Cm, ac4Cm, hm5C, C+, ho5C, hm5Cm, m2,7G, oQ, m22G, m1G, Gm, yW, Gr(p), m2G, m22Gm, gluQ, mimG, preQ1base, m1Gm, Q, yW-86, yW-72, galQ, imG2, manQ, OHyW, G+, m2Gm, imG-14, m227G, yW-58, m7G, preQ0, o2yW, preQ1, imG, m2,7Gm, f5U, m5s2U, s4U, m1acp3Y, cm5s2U, m5U, D, m5D, tm5U, cmnm5Um, f5Um, mcm5U, ncm5Um, nm5s2U, ncm5U, cm5U, cmnm5s2U, cmo5U, m3Y, m3U, Um, acp3Um, cmnm5se2U, m3Um, mcmo5U, nm5se2U, ho5U, f5s2U, mcm5s2U, chm5U, mo5U, acp3U, mnm5se2U, mcm5Um, mchm5U, cmnm5U, m1Y, tm5s2U, se2U, s2U, Ym, f5se2U, mnm5s2U, nm5U, s2Um, Y, m5Um, mnm5U, acp3D, acp3Y, inm5U, inm5Um, inm5s2U, ncm5s2U, nchm5U, mchm5Um, ges2U, cmnm5ges2U, mnm5ges2U, nm5ges2U, cnm5U and mcmo5Um.

The signal calculation part 330 may calculate an RNA modification signal value by using high-depth epitranscriptome sequencing data sequenced from a specifically produced sequencing library that detects specific chemical modifications of RNA (e.g., m⁶A, m⁵C, Ψ, I, ac⁴C, Nm, m⁶Am, m¹A, m⁷G, hm⁵C, etc.) or high-depth transcriptome sequencing data based on a specific sequencer (e.g., a sequencer from Oxford Nanopore) that can directly read RNA chemical modification information from the transcriptome genome sequencing library.

For example, it is possible to utilize high-depth cell-free nucleic acid sequencing data obtained by producing a transcriptome bisulfite sequencing library and sequencing the same with an Illumina sequencer to detect m⁵C, which is a type of RNA methylation modification, or it is possible to utilize high-depth cell-free nucleic acid sequencing data obtained by sequencing a whole genome sequencing library with an Oxford Nanopore sequencer.

In more detail, the signal calculation part 330 obtains RNA chemical modification sites and sites where RNA chemical modification does not occur from high-depth epitranscriptome sequencing data or a transcriptome sequencing library based on a specific sequencer, and filters out single nucleotide polymorphism (SNP) sites known in the past or removes inappropriate RNA modification sites by considering base quality, mapping quality, depth and the like on the sequencing read. Thereafter, the RNA chemical modification signals of a patient with a disease and a normal individual are compared to select an RNA chemical modification site where the pattern is significantly different in the patient with a disease, and the level of RNA chemical modification at the corresponding site may be calculated as an RNA signal value. In addition, the signal calculation part 330 may reflect the level of RNA chemical modification at the corresponding site, sequencing read information and the like to the RNA signal value.

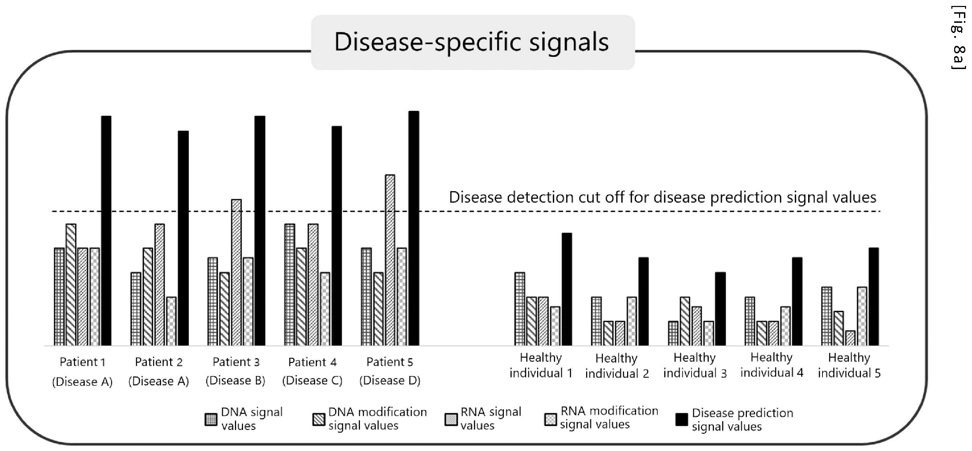

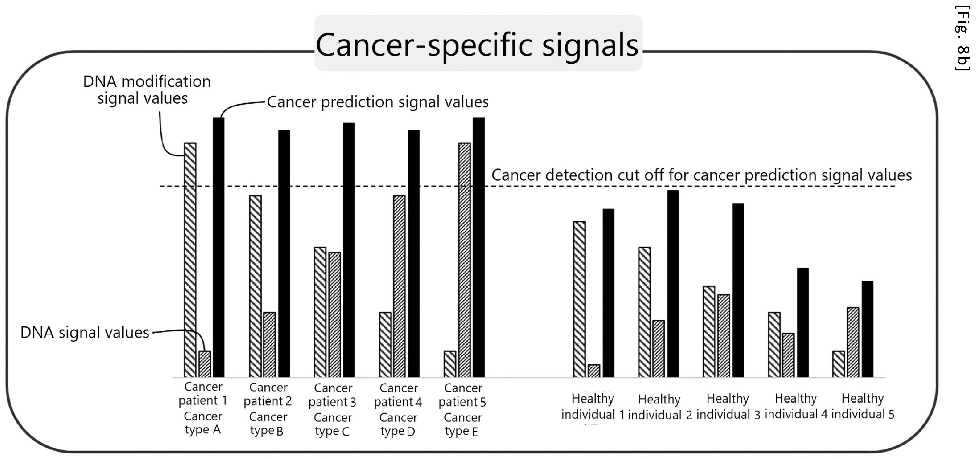

FIG. 8 is a diagram for describing a disease prediction signal value according to one embodiment of the present invention.

Referring to FIG. 8, the prediction signal value generation part 340 may generate a disease prediction signal value by processing at least one disease-specific signal value. The prediction signal value generation part 340 may generate a disease prediction signal value by processing the disease-specific signal value through standardization, one-hot encoding, low-quality data filtering, random partial selection or partial selection according to specific criteria. For example, the prediction signal value generation part 340 may generate a disease prediction signal value according to the type of a target disease by processing a DNA signal value and a DNA modification signal value.

In one embodiment, the prediction signal value generation part 340 may process a plurality of disease prediction signal values into vector data to generate a disease prediction signal value. For example, the prediction signal value generation part 340 may express a plurality of disease prediction signal values a, b, c and d as vector values by specifying each position. More specifically, the prediction signal value generation part 340 may select and process a specific disease-specific signal value according to the type of a target disease to generate a disease prediction signal value.

In one embodiment, the prediction signal value generation part 340 may generate a disease prediction signal value according to the type of a target disease. As described above, the prediction signal value generation part 340 may select a disease-specific signal value to be used according to the type of a target disease, and process the same to generate a disease prediction signal value.

In the example of FIG. 8A, the prediction signal value generation part 340 may generate a disease prediction signal value by combining a DNA signal value, a DNA modification signal value, an RNA signal value and an RNA modification signal value for a disease called A.

In the example of FIG. 8B, the prediction signal value generation part 340 may generate a cancer prediction signal value by combining the DNA signal value and the DNA modification signal value calculated for colon cancer, and may generate a cancer prediction signal value by combining the DNA modification signal value and the RNA signal value calculated for pancreatic cancer.

In more detail, each disease-specific signal value produced may be different depending on the type of a target disease, and accordingly, the prediction signal value generation part 340 may process the disease-specific signal value to generate a disease prediction signal value depending on the type of a target disease. Accordingly, the prediction signal value generation part 340 may detect various types of diseases by various combinations of DNA signal values, DNA modification signal values, RNA signal values and RNA modification signal values.

The prediction model generation part 350 may generate a disease prediction model that detects whether a disease occurs in the input blood by using labeling information related to blood and a disease prediction signal value.

More specifically, the prediction model generation part 350 may create a disease prediction model through learning by using labeling information related to blood and a disease prediction signal value generated through the corresponding blood. Here, creation through learning means that a predefined operation rule or an artificial intelligence model set to perform a desired characteristic (or purpose) is created by training an artificial intelligence model using a plurality of learning data by a learning algorithm. Such learning may be performed in the device itself on which the artificial intelligence according to the present disclosure is performed, or may be performed through a separate server and/or system. Examples of the learning algorithm include supervised learning, unsupervised learning, semi-supervised learning or reinforcement learning, but are not limited to the examples described above.

The artificial intelligence model may be configured by a plurality of neural network layers. Each of the plurality of neural network layers has a plurality of weight values, and performs neural network operations through operations between the operation results of the previous layer and the plurality of weights. The plurality of weights of the plurality of neural network layers may be optimized by the learning results of the artificial intelligence model. For example, the plurality of weights may be updated such that the loss value or cost value obtained from the artificial intelligence model is reduced or minimized during the learning process.

The artificial neural network may include a deep neural network (DNN), such as a convolutional neural network (CNN), a deep neural network (DNN), a recurrent neural network (RNN), a restricted Boltzmann machine (RBM), a deep belief network (DBN), a bidirectional recurrent deep neural network (BRDNN) or deep Q-networks (DQN), but is not limited to the above examples. For example, learning algorithms such as linear discriminant analysis (LDA), logistic regression, probit regression, naive bayes, bayes network, partial least squares, k-nearest neighbor, genetic programming, decision tree, random forest and support vector machine may also be included.

Meanwhile, the artificial intelligence model used in the disclosed embodiments may be implemented in various embodiments depending on the manufacturer of an electronic device or the user of an electronic device, and is not limited by the examples above.

The disease prediction part 360 may receive data on blood extracted from a subject and detect whether the subject has developed a disease through a disease detection model. For example, by inputting high-depth cell-free nucleic acid sequencing data generated based on cell-free nucleic acid extracted from the blood of a specific subject into a disease detection model generated through the prediction signal value generation part 340, it is possible to detect whether the subject has developed a disease.

Meanwhile, the blood of the subject to be tested for disease onset in the disease prediction part 360 should not be utilized in a disease detection model pre-generated by the prediction signal value generation part 340, and this is to prevent overfitting of the disease detection model.

For example, a library set may be produced through the library production part 310 by using cell-free nucleic acids extracted from the blood of a subject, the library set may be sequenced through the sequencing part 320, high-depth cell-free nucleic acid data may be mapped to a reference genome through the signal calculation part 330, and a disease prediction signal value may be generated from the mapped sequencing data through the prediction signal value generation part 340. In addition, it is possible to predict whether a subject has a disease and the type of disease through the disease prediction signal value pre-generated through the disease prediction part 360 and the generated disease prediction model.

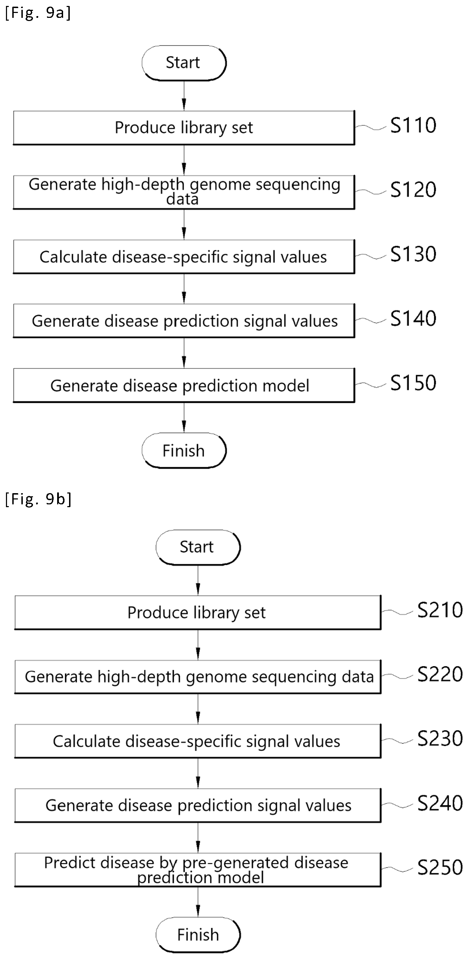

FIG. 9 is a diagram for describing the sequence of a disease detection method based on high-depth cell-free nucleic acid sequencing according to one embodiment of the present invention.

FIG. 9A is a schematic flowchart of a method for generating a disease prediction model based on high-depth cell-free nucleic acid sequencing.

Referring to FIG. 9A, the method for detecting a disease based on high-depth cell-free nucleic acid sequencing may produce a library set including a whole genome sequencing library, a whole epigenome sequencing library and a transcriptome sequencing library based on cell-free nucleic acids extracted from blood through the library production part 310 S110.

The method for detecting a disease based on high-depth cell-free nucleic acid sequencing may generate high-depth cell-free nucleic acid sequencing data by sequencing a library set through the sequencing part 320 S120.

The method for detecting a disease based on high-depth cell-free nucleic acid sequencing may map high-depth cell-free nucleic acid sequencing data to a reference genome through the signal calculation part 330, specify the location of the high-depth cell-free nucleic acid sequencing data on a genome, and generate at least one disease-specific signal value based on the mapped high-depth cell-free nucleic acid sequencing data S130.

The method for detecting a disease based on high-depth cell-free nucleic acid sequencing may generate a disease prediction signal value by processing at least one disease-specific signal value through the prediction signal value generation part 340 S140.

Referring to FIG. 9A, the method for detecting a disease based on high-depth cell-free nucleic acid sequencing may generate a disease prediction model that detects whether a disease occurs and the type of a disease in input blood by using labeling information related to blood and a disease prediction signal value through the prediction model generation part 350 S150.

FIG. 9B is a schematic flowchart of a method for predicting a disease by using a disease prediction model generated based on high-depth cell-free nucleic acid sequencing.

Referring to FIG. 9B, the method for detecting a disease based on high-depth cell-free nucleic acid sequencing may detect whether a disease has occurred and the type of a disease in input blood by using labeling information related to blood, a disease prediction signal value and a pre-generated disease prediction model through the disease prediction part 360 S250.

Although the present invention has been described above with reference to preferred embodiments thereof, it will be understood by those skilled in the art that various modifications and changes may be made to the present invention without departing from the concept and scope of the present invention as set forth in the claims below.

**[Explanation of Reference Numerals]**

| | | | |
|---|---|---|---|
| 100: | Disease detection system based on high-depth cell-free nucleic acid sequencing | | |
| 110: | User terminal | | |
| 130: | Disease detection apparatus based on high-depth cell-free nucleic acid sequencing | | |
| 150: | Database | | |
| 210: | Processor | 230: | Memory |
| 250: | User input/output part | 270: | Network input/output part |
| 310: | Library production part | | |
| 320: | Sequencing part | | |
| 330: | Signal calculation part | 340: | Prediction signal value generation part |
| 350: | Prediction model generation part | 360: | Disease prediction part |

## Claims

1. An apparatus for early disease detection based on high-depth cell-free nucleic acid sequencing, comprising:
a library production part configured to produce a library set comprising a whole genome sequencing library, a whole epigenome sequencing library and a transcriptome sequencing library based on cell-free nucleic acids extracted from blood;
a sequencing part configured to generate high-depth cell-free nucleic acid sequencing data by sequencing the library set at a high depth of 10 or more;
a signal calculation part configured to map the high-depth cell-free nucleic acid sequencing data to a reference genome so as to specify a location of the high-depth cell-free nucleic acid sequencing data on a genome, and calculate at least one disease-specific signal value based on the mapped high-depth cell-free nucleic acid sequencing data;
a prediction signal value generation part configured to generate a disease prediction signal value by processing the at least one disease-specific signal value; and
a prediction model generation part configured to generate a disease prediction model that detects whether a disease occurs and the type of a disease for input blood by using labeling information related to blood and the disease prediction signal value.

2. The apparatus of claim 1, wherein the blood is extracted from individuals without disease, individuals with a history of a specific disease but currently without disease, patients in the early, middle and late stages of a specific disease, and patients with a relapse of a specific disease.

3. The apparatus of claim 1, wherein the signal calculation part calculates the disease-specific signal value based on one or more of a DNA signal value, a DNA modification signal value, an RNA signal value and an RNA modification signal value.

4. The apparatus of claim 1, wherein the prediction signal value generation part processes a plurality of disease-specific signal values into vector data to generate the disease prediction signal value.

5. The apparatus of claim 1, wherein the prediction signal value generation part generates the disease prediction signal value according to the type of a target disease.

6. The apparatus of claim 1, further comprising:
a disease prediction part configured to receive blood data extracted from a subject and detect whether the subject has a disease and the type of a disease through the disease prediction model.

7. The apparatus of claim 3, wherein the DNA signal value comprises one or more of an amount of cell-free DNA extracted from blood, a set of base sequences at and around a mutation site, a gene copy number variation, a chromosomal aneuploidy, a disease-specific mutation, a genome sequence of a detected pathogen, a distribution of fragment lengths, sequence information around fragment ends, an individual disease-specific nucleosome distribution obtained from location information at fragment ends, and a ratio of jagged ends.

8. The apparatus of claim 3, wherein the DNA modification signal value comprises one or more position-specific modification levels of 1mA, 1mHyp, 3mA, 3mHyp, 4mA, 6mA, 7mA, 7mHyp, 9mA, 9mHyp, 6hmA, m2A, ncm6A, xI, m2C, e3C, 3mC, εC, 4mC, 5caC, 5fC, 5hmC, 5mC, 1mG, 2mG, 3mG, m6G, 7mG, CEG, M22G, N(2),3-εG, 1,N(2)-εG, ADG, 8oxoG, dU, dhpdU, 5-NedU, DHdU, 5-NeOmdU, 5caU, dhpUra, 5hmU, 5fU, base J, diHT, 1mT, 3mT, 4-meT and putThy.

9. The apparatus of claim 3, wherein the RNA signal value comprises one or more of an expression level of individual disease-specific genes, an expression level of fusion genes and a degree of alternative splicing.

10. The apparatus of claim 3, wherein the RNA modification signal value comprises one or more position-specific modification levels of ms2m6A, io6A, m6t6A, hn6A, Ar(p), m1I, m1Am, m1Im, m2A, m62A, ms2i6A, ms2t6A, i6A, ms2hn6A, m6Am, m1A, m6A, I, g6A, m62Am, ac6A, ms2io6A, Am, Im, t6A, m8A, m2,8A, ct6A, hm6A, f6A, ms2ct6A, ht6A, msms2i6A, m5Cm, m4C, m42C, m5C, f5C, m4Cm, m3C, m42Cm, ac4C, Cm, s2C, k2C, f5Cm, ac4Cm, hm5C, C+, ho5C, hm5Cm, m2,7G, oQ, m22G, m1G, Gm, yW, Gr(p), m2G, m22Gm, gluQ, mimG, preQ1base, m1Gm, Q, yW-86, yW-72, galQ, imG2, manQ, OHyW, G+, m2Gm, imG-14, m227G, yW-58, m7G, preQ0, o2yW, preQ1, imG, m2,7Gm, f5U, m5s2U, s4U, m1acp3Y, cm5s2U, m5U, D, m5D, tm5U, cmnm5Um, f5Um, mcm5U, ncm5Um, nm5s2U, ncm5U, cm5U, cmnm5s2U, cmo5U, m3Y, m3U, Um, acp3Um, cmnm5se2U, m3Um, mcmo5U, nm5se2U, ho5U, f5s2U, mcm5s2U, chm5U, mo5U, acp3U, mnm5se2U, mcm5Um, mchm5U, cmnm5U, m1Y, tm5s2U, se2U, s2U, Ym, f5se2U, mnm5s2U, nm5U, s2Um, Y, m5Um, mnm5U, acp3D, acp3Y, inm5U, inm5Um, inm5s2U, ncm5s2U, nchm5U, mchm5Um, ges2U, cmnm5ges2U, mnm5ges2U, nm5ges2U, cnm5U and mcmo5Um.

11. The apparatus of claim 1, wherein the sequencing part utilizes two or more sequencers to sequence a library set.

12. A method for detecting a disease based on high-depth cell-free nucleic acid sequencing, the method comprising:
producing a library set comprising a whole genome sequencing library, a whole epigenome sequencing library, a transcriptome sequencing library and an epitranscriptome sequencing library based on cell-free nucleic acids extracted from blood;
generating high-depth cell-free nucleic acid sequencing data by sequencing the library set at a high depth;
mapping the high-depth cell-free nucleic acid sequencing data to a reference genome so as to specify a location of the high-depth cell-free nucleic acid sequencing data on a genome, and calculating at least one disease-specific signal value based on the mapped high-depth cell-free nucleic acid sequencing data;
generating a disease prediction signal value by processing the at least one disease-specific signal value; and
generating a disease prediction model by using labeling information related to blood and the disease prediction signal value.
